# EUROPEAN PATENT APPLICATION

(11) **EP 4 666 942 A1**
(43) Date of publication of application: **24.12.2025**
(21) Application number: 24183781.4
(22) Date of filing: 21.06.2024
(51) Int. Cl.: A61B 5/103, A61B 5/11, G06V 40/20

(54) **METHOD AND SYSTEM FOR MEASURING AT LEAST ONE SPATIAL, TEMPORAL OR SPATIOTEMPORAL GAIT PARAMETER**

(71) Applicant: Universität Bern, 3012 Bern (CH)
(72) Inventor: SINGLE, Michael, 3008 Bern (CH); NEF, Tobias, 3008 Bern (CH); BRUHIN, Lena, 3008 Bern (CH); KRUMMENACHER, Nic, 3008 Bern (CH); BARCIA, Antonio, 3008 Bern (CH)
(74) Representative: Braunpat AG

(57) **Abstract**

The present invention relates to a method (100) for measuring at least one spatial, temporal or spatiotemporal gait parameter, comprising: Acquiring seismic signals by at least three time-synchronized seismographs positioned to span the floor of a measurement zone during walking of at least one walker on the floor of the measurement zone (101); Receiving and processing the seismic signals by an analysis device to obtain a combined signal representing an aggregate signal of the seismographs (102); Identifying and extracting footsteps events from the seismic combined signal, wherein each footstep event corresponds to the contact between a walker's foot and the floor in the measurement zone (103); and Calculating at least one gait parameter from the combined signal and the extracted footstep events (104). In addition, the present invention relates to a system for measuring at least one spatial, temporal or spatiotemporal gait parameter (200),

## Description

### Technical Field

The present invention relates to the field of gait analysis and more specifically to methods and systems for measuring spatial, temporal, or spatiotemporal gait parameters. The invention concerns more specifically a method and a system designed for measuring at least one spatial, temporal or spatiotemporal gait parameter. The gait parameters are critical for applications in clinical diagnostics, rehabilitation, sports science, and ergonomic studies, providing valuable insights into an individual's locomotion characteristics, identifying abnormalities, and assessing the effectiveness of therapeutic interventions.

### Background of the invention

Gait analysis, the systematic study of walking, serves as a medical procedure for evaluating and interpreting human locomotion. Typically, this type of locomotion analysis is performed through assessments based on human observation or with the help of advanced sensor technology. Specifically, changes in locomotion patterns are examined over time to identify potential signs of abnormalities that may indicate underlying health issues or the presence of injuries. Observing such changes has been demonstrated to be valuable in detecting conditions associated with aging and neurodegenerative diseases.

Early detection of symptoms related to these conditions enables prompt treatment of the affected patients, which helps to preserve their independence and quality of life. In clinical practice, observation-based movement assessments are carried out by specialists. However, these assessments are limited by the variability and inconsistency caused by subjective biases, which are a matter of concern, given that the assessments directly influence the determination of patient-specific interventions and medical treatments. Furthermore, such assessments only capture a single moment in time, potentially complicating the early diagnosis of emerging diseases or the tracking of disease progression. Repeated assessments over time may be necessary to determine changes in an individual's health status. However, frequent medical visits or long-term stays at expensive healthcare facilities are currently required in standard practice, ultimately contributing to the bottleneck in performing long-term assessments.

This limitation can be overcome using advanced sensor technology known from the state of the art through the incorporation of health monitoring in smart home solutions, enabling older adults to remain in their homes and thereby reducing the need for institutional care. Typically characterized as being either ambient or wearable, these sensor technologies are used to quantify and extend the continuity of the locomotion analysis by deriving spatiotemporal gait parameters. In terms of ambient sensors, pressure-sensitive walkways are a conventional clinical reference system used to measure forces while walking. However, despite their accuracy, these walkways are limited by their measurement area (because of their fixed length and width) and high acquisition costs. Motion capture systems are a popular ambient sensor technology that can be used to assess human locomotion by tracking markers placed on key anatomical features. These markers are used to track positions and calculate velocity, acceleration, and joint angles, but they often require expertise in their placement and data interpretation. Moreover, privacy concerns, physical challenges, and possible occlusion limit the practical application in patients' homes.

In contrast, wearable inertial measurement units, attached to the individual's body, offer an affordable and portable solution for assessing acceleration and rotation (e.g., they can be worn on the feet to measure gait). However, they are prone to time-induced drift and need to be calibrated, and the extraction of clinical features like gait information requires algorithmic processing. Furthermore, their use in uncontrolled settings (e.g., long-term measurements at home), particularly by older adults or patients with neurodegenerative conditions, can be challenging owing to low acceptance and compliance rates, potentially making them difficult to use for longitudinal studies. Thus, there is a need for a robust, unintrusive, and contactless measurement system capable of monitoring human gait over prolonged periods in clinical and residential settings that requires minimal user engagement and is cost-effective.

### Summary of the invention

Thus, the object of the present invention is to propose a novel method and a novel system for measuring at least one spatial, temporal or spatiotemporal gait parameter, with which the above-described drawbacks of the known methods and systems are completely overcome or at least greatly diminished.

According to the present invention, these objects are achieved in particular through the elements of the independent claims. Further advantageous embodiments follow moreover from the dependent claims and the description.

In particular, the objects of the present invention are achieved in a first aspect by a method for measuring at least one spatial, temporal or spatiotemporal gait parameter, comprising:
a. Acquiring seismic signals by at least three time-synchronized seismographs positioned to span the floor of a measurement zone during walking of at least one walker on the floor of the measurement zone;
b. Receiving and processing the seismic signals by an analysis device to obtain a combined signal representing an aggregate signal of the seismographs;
c. Identifying and extracting footsteps events from the combined signal, wherein each footstep event corresponds to the contact between a walker's foot and the floor in the measurement zone;
d. Calculating at least one gait parameter from the combined signal and the extracted footstep events.

Thanks to the method of the present invention, it is possible to perform robust, unintrusive, and contactless measurements and monitoring of human gait parameters over prolonged periods in clinical and residential settings that requires minimal user engagement and in a cost-effective manner. The inventive method allows a continuous assessment of human gait parameters (i.e., a gait analysis) and does not require any supervision to perform a measurement. The circumstance that no human operator is needed to perform an active measurement and that it is not based on wearable devices reduce potential erroneous measurements due to incorrect usages. The continuous aspect allows medical experts to obtain a more holistic overview of a measured person's health status and thus allows them to base their decision-making on a more informed basis. Compared to methods based on the use of pressure-sensitive walkways, the measurement zone of the present invention can be defined with more flexibility thus allowing for the implementation of the inventive method in completely different environments. Finally, the use of at least three seismographs allows for locating the footstep events through triangulation.

Advantageously, at least one measured gait parameter is selected from: step count, step time, stride time, cycle time, stance time, swing time, ambulation time, walking time, cadence, velocity, speed, footedness step length, stride length, walking distance, gait symmetry, and walking direction.

In a first preferred embodiment of the first aspect of the present invention, the method further comprises a step of temporally aligning the signals from the seismographs for generating the combined signal. Thanks to the alignment and the combination of the signals acquired by the at least three seismographs, it is possible to improve the robustness of the steps extraction and detection.

In another preferred embodiment of the fist aspect of the present invention, the method further comprises a step of localization of footstep events by comparing signal arrival times at the seismographs. Advantageously, the localization of footstep events is determined by using an estimation of the wave propagation velocity in the measurement zone. This estimation is advantageously based on a calibration step that can made before or after the present method has been performed.

In a further preferred embodiment of the first aspect of the present invention, the method further comprises a step of applying morphological filtering to the combined signal for noise reduction.

In yet another preferred embodiment of the first aspect of the present invention, the method further comprises a step of determining an average walking direction between footstep events by calculating a walking direction vector based on location and time differences of the footstep events.

In a further preferred embodiment of the first aspect of the present invention, footedness is determined by comparing footstep event locations with the average walking direction.

In another preferred embodiment of the present invention, the method further comprises a step of calculating for each footstep event a time difference between the contact of the heel of the walker and the floor and the contact between the toe of the walker and the floor. This allows for calculating the heel-to-toe contact time, which is part of the stance phase of the gait cycle. It provides valuable information about the temporal aspects of a person's walking pattern and can be used to assess various aspects of gait, including balance, coordination, and potential abnormalities.

In yet another preferred embodiment of the first aspect of the present invention, the method further comprises a step of extracting a signature signal for each walker identifying the walker and storing signature signals in a database. This allows for building a database comprising gait signature signal from different individuals and to compare the gait signals from an individual over time and/or to compare the gait signal from different individuals.

In still another preferred embodiment of the first aspect of the present invention, at least two walkers are present in the measurement zone, and the combined signal is decomposed into person-specific signals using signature signals to measure walker-specific gait parameters. Thanks to this, it is possible to measure gait parameters of one or more individuals even if more than one walker is present in the measurement zone.

In a further preferred embodiment of the first aspect of the present invention, the method further comprises a step of calculating walking energy for each footstep event by combining the signals of the corresponding footstep event acquired by the at least three seismographs. Assessing walking energy is important in diagnosing and managing conditions like Parkinson's disease, cerebral palsy, and other mobility impairments. It helps in the design of rehabilitation programs and evaluating the effectiveness of interventions such as prosthetics, orthotics, and physical therapy.

In a second aspect, the goals of the present invention are also achieved by a system for measuring at least one spatial, temporal or spatiotemporal gait parameter, comprising:
- At least three seismographs positioned to span the floor of a measurement zone and configured to acquire seismic signals during walking of at least one walker in the measurement zone;
- An analysis device coupled to receive the seismic signals from the seismographs and configured to:
   ▪ Process the received seismic signals to obtain a combined signal representing the aggregate signal of the seismographs; and
   ▪ Identify and extract footsteps events from the combined signal, wherein each footstep event corresponds to the contact between a walker's foot and the floor in the measurement zone;
- A processor configured to calculate at least one, spatial, temporal or spatiotemporal gait parameter from the combined signal and the extracted footstep events; and
- A memory device coupled to store the calculated gait parameters.

Such a system allows performing the inventive method of the present application. It allows in particular to perform robust, unintrusive, and contactless measurements and monitoring of human gait parameters over prolonged periods in clinical and residential settings that requires minimal user engagement and in a cost-effective manner. The system allows a continuous assessment of human gait parameters (i.e., a gait analysis) and does not require any supervision to perform a measurement. The circumstance that no human operator is needed to perform an active measurement reduces potential erroneous measurements due to incorrect usages. The continuous aspect allows medical experts to obtain a more holistic overview of a measured person's health status and thus allows them to base their decision-making on a more informed basis.

In a first preferred embodiment of the second aspect of the present invention, the analysis device is further configured to apply morphological filtering to the combined signal for noise reduction.

In another preferred embodiment of the second aspect of the present invention, the processing unit is further configured to determine an average walking direction between footstep events and calculate footedness based on location differences.

In a further aspect, the goals of the present invention are also achieved by a computer program product comprising instructions to cause the analysis device of the system of the present invention to execute the steps of the method of the present invention.

In yet a further aspect, the goals of the present invention are also achieved by a computer-readable medium having stored thereon the computer program product of the present invention.

### Brief description of the drawings

Figure 1 is workflow chart presenting a method for measuring at least one spatial, temporal or spatiotemporal gait parameter according to one embodiment of the present invention;
Figure 2 is a schematic illustration of a system for measuring at least one spatial, temporal or spatiotemporal gait parameter according to one embodiment of the present invention;
Figure 3 illustrates the experimental setup of the comparative study presented here;
Figure 4 illustrates the stages involved in the seismograph-based step extraction method according to one embodiment of the present invention;
Figure 5 shows the dampened seismic signals acquired by the seismographs alongside their aggregated signal;
Figure 6 Illustrates the effect of the morphological operations applied on a seismographic signal, together with the intermediate results of the CTF (compound top-hat) filter;
Figure 7 presents plots of the linear relationships of the gait parameters between measurements assessed using the pressure-sensitive walkway and the seismographs;
Figure 8 shows the distributions of step lengths and velocities across the different ambulation experiments;
Figure 9 presents plots of the linear relationships of gait parameters between measurements assessed using the pressure-sensitive walkway and the seismographs; and
Figure 10 presents Bland-Altman plots to demonstrate agreement of matching gait parameters between the seismograph and the walkway measurements.

### Detailed description of a preferred embodiment

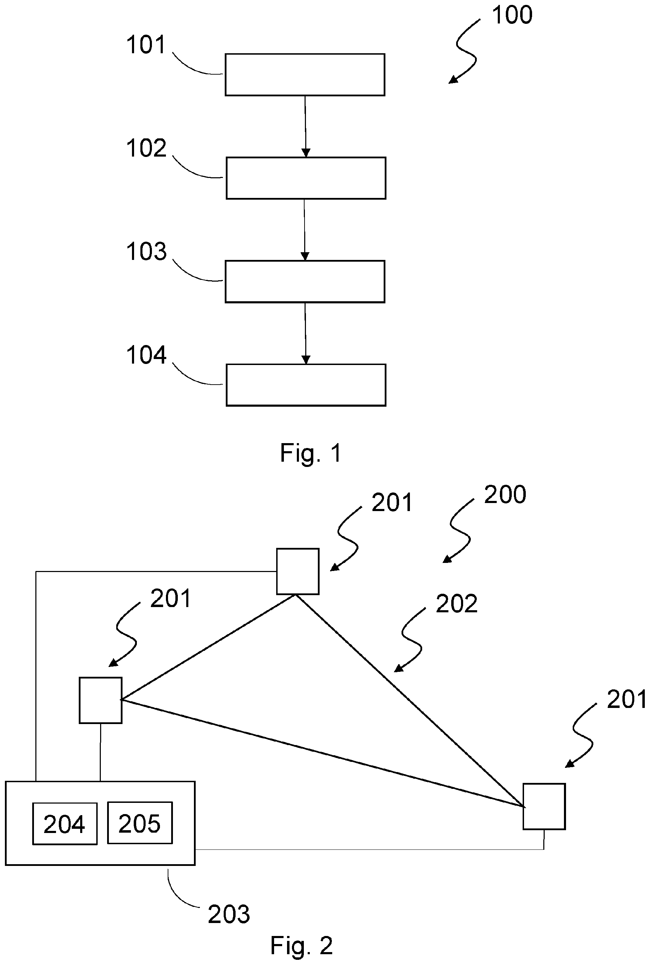

Figure 1 illustrates a method 100 for measuring at least one spatial, temporal or spatiotemporal gait parameter according to one embodiment of the present invention. In step 101, seismic signals are acquired by at least three time-synchronized seismographs positioned to span the floor of a measurement zone during walking of at least one walker on the floor of the measurement zone 202 (see Fig. 2). In step 102, the seismic signals are received and processed by an analysis device to obtain a combined signal representing an aggregate signal of the seismographs. In step 103, footsteps events are identified and extracted from the combined signal, wherein each footstep event corresponds to the contact between a walker's foot and the floor in the measurement zone. In step 104, at least one gait parameter is calculated from the combined signal and the extracted footstep events.

Figure 2 is a schematic illustration of a system 200 for measuring at least one spatial, temporal or spatiotemporal gait parameter according to one embodiment of the present invention. As illustrated, three seismographs 201 are positioned to span the floor of a measurement zone 202 and are configured to acquire seismic signals during walking of at least one walker in the measurement zone. The system 200 comprises also an analysis device 203 coupled to receive the seismic signals from the seismographs and configured to process the received seismic signals to obtain a combined signal representing the aggregate signal of the seismographs 201 and to identify and extract footsteps events from the combined signal, wherein each footstep event corresponds to the contact between a walker's foot and the floor in the measurement zone 202. The analysis device comprises advantageously a processor 204 configured to calculate at least one, spatial, temporal or spatiotemporal gait parameter from the combined signal and the extracted footstep events and a memory device 205 to store the calculated spatiotemporal gait parameters.

Advantageously, the seismographs are moving coil geophone sensors. The sensors are in particular advantageously configured to measure low-frequency shear waves at a natural frequency of up to 4.5 Hz and at a sensitivity level in the circuit of approx. 28.8 V/m/s. The geophone sensors are mounted to ensure that they are properly aligned with the ground and oriented vertically and are directly integrated into the signal processing unit via a wired connection with the analysis device 203.

### Experimental results of a comparative study

In order to show the capability of the method and of the system of the present application in comparison to previously known methods and systems a comparative study has been performed, using off-the-shelf products. A convenience sample of 50 healthy individuals ranging in age from 19 to 71 years (mean, 32.86 years; SD, 11.08 years) was recruited for an observational, cross-sectional study. The sample was gender-balanced, encompassing 26 women and 24 men. Participants were eligible for inclusion if they were at least 18 years of age, and exhibited no walking impairments that could affect their regular daily activities. The study protocol was explained to each participant verbally, and written informed consent was obtained prior to participation. This study was conducted in a home-like instrumented apartment.

Each participant undertook four predefined ambulation experiments (i.e., two free walks and two walks with a metronome, one to a fast beat and one to a normal beat) by walking over a pressure sensitive walkway defining the measurement zone and performing the Timed up and go test (TUG) according to the following protocol: The procedure begins with the participant seated comfortably in a standard armchair, facing a clearly marked line 3 meters away on the floor. Instructions are then given to the participant as follows: "Upon the signal 'Go,' you will be required to: 1. Stand up from the chair; 2. Walk at your natural pace to the marked 3-meter line on the floor; 3. Execute a turn; 4. Return to the chair at your natural walking speed; 5. Sit down once more." The timing of this assessment starts with the signal "Go" and stops when the participant is seated again. Prior to initiating a measurement, participants were provided detailed instructions on the execution of the forthcoming ambulation. To ensure that the participants understood the experiment instructions, they were asked to do a test run before each ambulation experiment. The free-walking experiments were recorded twice and conducted at a self-regulated pace, thereby allowing participants to choose any pace that felt comfortable and natural to them. The remaining two ambulation experiments were conducted at a prespecified pace using a metronome.

During the measurement, participants were requested to traverse the walkway without wearing their shoes, thereby eliminating the dampening effect of footwear as a confounding variable. The influence of any further material-related damping effects concerning the seismic amplitudes was not taken into effect, as preliminary measurements before the present study showed distinct amplitudes associated with footstep events in the recorded seismic signals. To further reduce external influences that might have significantly affected the quality of the measured data, particularly concerning the seismic measurements, no person other than the patient being measured was in the vicinity of the sensor devices or walking around. For consistency, the starting position of every recorded walk was specified at the leftmost end of the pressure-sensitive walkway. Participants were requested not to leave the active area of the walkway at any point during the measurement to ensure the quality of the recorded data.

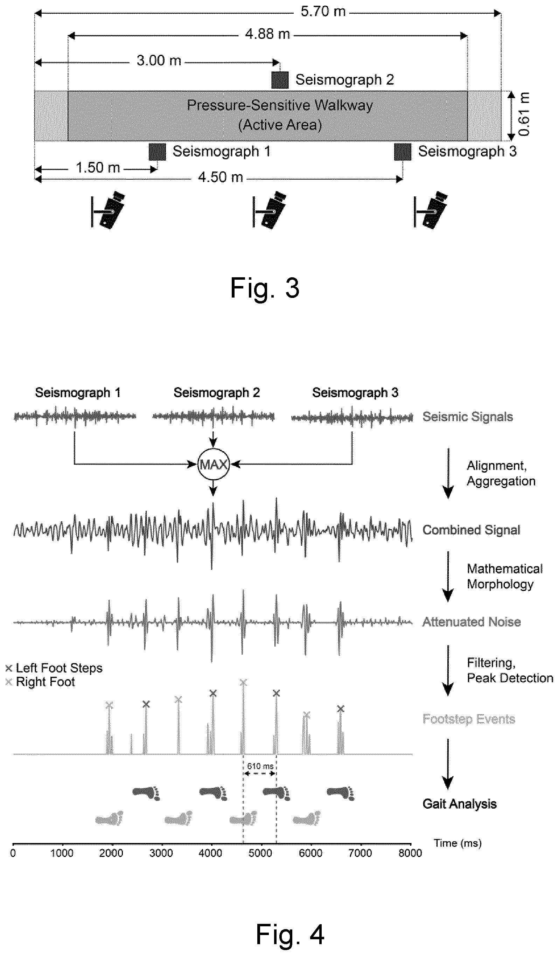

Three seismographs (RS-4D, Raspberry Shake S.A, Alto Boquete, Panama) and two reference systems, namely a pressure- sensitive walkway (GAITRite^{®}, CIR Systems Inc. Clifton, NJ 07012, USA) and a camera system (Qualisys QTM, Gothenburg, Sweden), were installed in the living room of the apartment to measure participant's gaits (Figure 3). The three seismographs were positioned on the floor along the walkway, maintaining a distance of 1.5 m between each of them. The first and last sensors were placed on the right side (in the direction of walking), while the second was situated on the left side. This sensor arrangement was used to facilitate the subsequent triangulation-based method steps. The camera system was calibrated to ensure an optimal range of angles, with each camera directed toward the walkway. The decision to incorporate a camera system alongside the pressure-sensitive walkway was motivated by the limitation of the walkway's measurement software, which was only capable of processing complete walks (i.e., participants walking from the starting point of the walkway to its end). This was especially crucial for accurately assessing timings in TUG tests, which involved a turning point on the walkway, resulting in crashing the measurement.

The three seismographs were employed to quantify the ground motion. These sensors utilize integrated geophones designed to measure vibrations at a low natural frequency of 4.5 Hz. This makes them ideally suited for accurately measuring human gait, considering that the effective natural frequency of human walking is below 2 Hz. The seismographs were configured to sample at a frequency of 100 Hz. A basic client-server application was developed to read the seismic samples from the RS-4D seismographs and transfer them via the network to a database. To measure spatial, temporal and spatiotemporal gait parameters, the pressure-sensitive walkway was used as a reference system. The specific walkway model employed had an active measurement length of 4.88 m and a width of 0.61 m. The data gathered from the system were sampled at a frequency of 80 Hz and analyzed using the GAITRite^{®} software (version 4.89H9). The following gait parameters were obtained from the analysis: average step time, average cycle time, total ambulation time, cadence per minute, average velocity, and average step length. In addition to the pressure-sensitive reference system, a marker less motion tracking system comprising 13 video cameras (further referred to as the camera system) was used to quantify the number of steps taken by the participants and determine the duration of their walking activities. All video recordings were set to a resolution of 1920 × 1080 pixels, sampled at a frequency of 85 Hz. The camera recordings were used to determine the timestamps of events associated with the TUG test at a frame-wise precision. The timings of the video frames were extracted by using the software Adobe Premiere Pro CC 2020 (version 14.3.1).

The fundamental principle of the inventive method originates from the premise that human footsteps can be likened to minor earthquakes, creating vibrations that propagate through the ground and can be detected by seismographs. Hence, identifying footsteps in seismic signals corresponds to determining timestamps of peaks that resemble earthquake seismic events. The particular stages of the seismograph-based step extraction of a preferred embodiment of the present method involved signal aggregation, mathematical morphology, filtering, and step extraction (Figure 4).

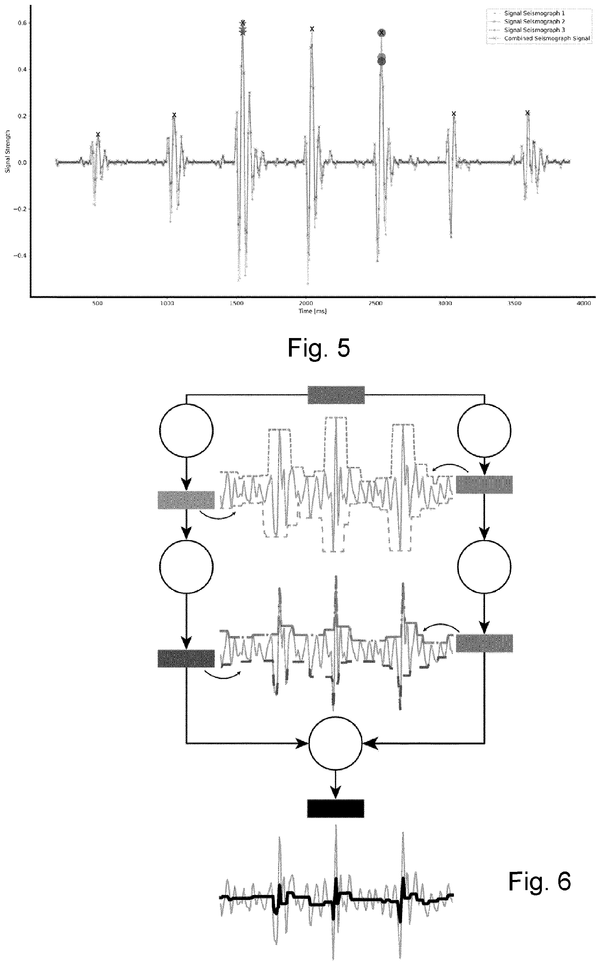

In a preferred embodiment, the measurements of the three seismographs were normalized and detrended by subtracting their mean values. The normalized signals were then temporally aligned and combined by applying a rolling maximum across their signals to enhance their overall quality. Such a maximum signal aggregation also has a positive influence on damping amplitude as it corrects degraded amplitudes, counteracting potential signal damping influences. Figure 5 qualitatively illustrates the influence of combining multiple seismic signals and how this can help to improve the robustness of the step detection. It is important to note that the maximal peak might not always originate from the same seismograph signal. This is clearly demonstrated in the examples highlighted by stars and circles: in the case of stars, the maximum peak is derived from the red seismic signal, whereas in the circles' case, it's from the blue signal. The present method is designed to always choose the peak with the highest amplitude from the combined signals, effectively counteracting signal-damping influences. This feature significantly enhances the robustness of the step-detection method.

A nonlinear reduction method based on mathematical morphologies was advantageously used to mitigate low-frequency noise in the seismographic signals. This type of noise attenuation can effectively identify and eliminate outliers, as well as extract key features, such as local peaks in time-series data. To attenuate the signal's noise, the average of its opening and closing was derived and then subtracted from the signal, which directly corresponds to the definition of the compound top-hat filters (CTF). The choice to employ Mathematical morphologies alongside CTF was grounded in their proven effectiveness in reducing low-frequency noise in microseismic measurements while still retaining significant seismic events, such as the footstep events targeted for extraction in the present study. The opening operation was implemented by first executing an erosion on the time series, then proceeding with a dilation, which helps remove sharp spikes in the data. Conversely, the closing operation was implemented by initially performing a dilation on the time series, followed by erosion, which helps to fill in sharp dips in the data. In these computations, a spherical structural element (SE) was used to dilate and erode the signal. The radius of the SE was chosen to 200 ms because this value corresponds to the duration of an average single footstep during human walking. A visualization of the morphological operations applied on a seismographic signal, together with the intermediate results of the CTF, is shown in Figure 6 in the form of a computation graph.

The resulting noise-attenuated signal was processed using two linear filters: a Hamming filter to boost peaks while simultaneously smoothing and a rectangular filter to eliminate potential closely adjoining double peaks. Both filters were employed using a 100 ms radius.

Footsteps were extracted by advantageously applying a thresholding-based peak detector to the filtered signal. Consequently, footstep events correspond to the timing of initial contacts of the foot (i.e., the timestamp of the extracted peak), which were used in the gait parameter calculation. The robustness of the peak detector was further increased by imposing the following constraints: requiring a minimum distance of 50 ms between two peaks and anticipating that the height of any potential peak would reside within the upper 95th percentile of the min-max normalized amplitudes. No additional amplitude enhancement procedures were performed to identify peaks, as the3noise-attenuated signals exhibited distinct characteristics.

In the gait analysis, the times of the extracted footstep events per ambulation were used to compute four temporal parameters (step time, cycle time, ambulation time, and cadence), one spatiotemporal parameter (velocity), and one spatial parameter (step length). The location of seismic events of interest (e.g., steps) was determined by comparing the time differences of event arrivals between time-synchronized stationary seismographs. To realize such a comparison, the seismographs were positioned to span an area enclosing the seismic events to be measured (Figure 3).

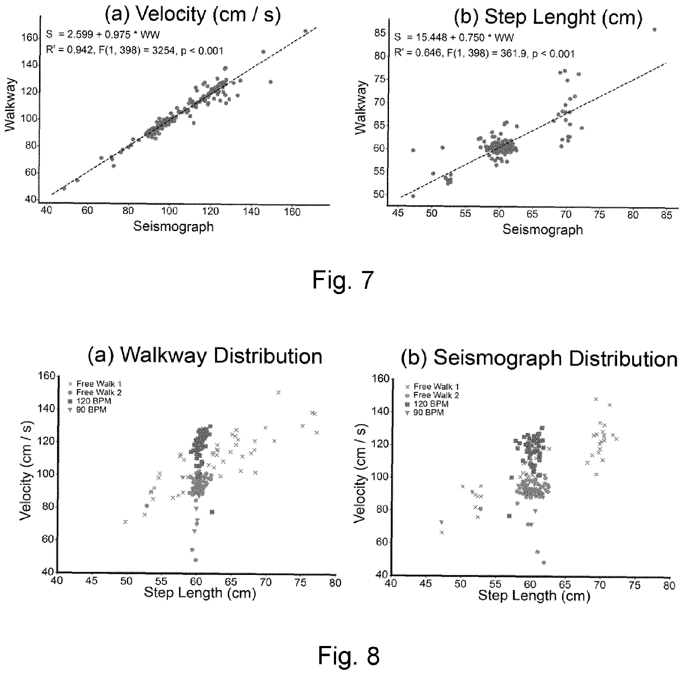

As can be seen in Fig. 7, the correlation of the approximated spatial gait parameters (i.e., velocity and step length) showed a strong positive correlation between measurements assessed with the seismographs and the pressure-sensitive walkway. In both plots of this Figure, the slope and inclination of the fitted linear model, the determination coefficients, and the F-scores are provided.

A visual observation of the seismic signal combined with the camera recordings was used to confirm that the initial peak of the seismic signal corresponds to the start of the TUG test and the last peak to the end of the test. As such, the same algorithm used for the gait analysis can be applied to extract the timings of the TUG test.

A comprehensive statistical comparison was conducted between the gait parameters computed using the seismograph-based method and the measurements acquired from the reference system (i.e., the pressure-sensitive walkway). More specifically, the two measurement systems were analyzed by comparing the calculated gait parameters from four different ambulation experiments per participant (i.e., Free Walk 1, Free Walk 2, Fast Walk, and Normal Walk). To establish an adequate comparison, the statistical analysis was exclusively confined to those seismographic measurements that occurred within the time frame of the walkway recordings.

Initially, descriptive statistics of all gait parameters were calculated for the seismographs and the walkway. Scatter plots of the distributions in step lengths and velocities were qualitatively compared between the two measurement methods.

Subsequently, the presence of a significant difference between the seismograph and the walkway measurement methods was evaluated by performing paired two-sample t-tests for all matching gait parameters. It was hypothesized that the mean values of matching gait parameters between the seismograph and walkway measurements were not significantly different (significance level alpha = 0.05).

Next, simple linear regression was applied to the mean values of the gait parameters from all ambulation experiments to analyze the relationship between the two measurement systems. The necessity of a linear regression model was validated by performing an F-test. This test incorporated the residual sum of squares for each paired set of measured and computed gait parameters. The coefficient of determination, R2, was calculated for each matching gait parameter to help clarify the error of the linear models. The correlation between the seismographic measurements and the walkway measurements of matching gait parameters was quantified by computing the Pearson correlation coefficient, r. To qualitatively interpret the correlation results, the terminology proposed by Schober et al. was applied (Schober, P., Boer, C. & Schwarte, L. A. Correlation coefficients: appropriate use and interpretation. Anesth. & analgesia 126, 1763-1768, DOI: https://doi.org/10.1213/ANE.0000000000002864 (2018)).

Finally, measured matching gait parameters were qualitatively examined using Bland-Altman plots. These plots were used to identify the presence of proportional and constant biases in the data within the bounds of the confidence intervals, as well as to determine the agreements between the matching gait parameters measured by the walkway and those measured by the seismographs.

The same statistical analysis procedures and visualization techniques were applied to report the results of the TUG test estimations and the ground-truth timings extracted from the video recordings. The second hypothesis stated that the mean values of the TUG times between the seismograph and camera system were not significantly different.

The distributions of step lengths and velocities showed similar characteristics for all conducted experiments (Figure 8). The mean velocity of the pressure-sensitive walkways was 103.0 cm/s (SD = 16.6 cm/s), ranging between 48.3 and 167.0 cm/s, and the mean velocity of the seismograph was 102.9 cm/s (SD = 16.5 cm/s) ranging between 48.4 and 165.5 cm/s. The mean step length of the pressure-sensitive walkway was 60.9 cm (SD = 3.9 cm), ranging between 49.8 and 86.2 cm, and the mean step length of the seismograph was 60.7 cm (SD = 4.2 cm), ranging between 47.1 and 82.9 cm.

In the present study, a total of 200 ambulation experiments were performed and recorded by 50 participants. The proposed seismograph method detected the exact number of performed steps (1577 of the 1577 steps). When employing a single seismograph-Seismograph 1, Seismograph 2, or Seismograph 3-the proposed method identified 1565, 1573, and 1567 steps out of 1577 steps, respectively. Among these ambulation experiments, the distribution of Free Walk 1 (crosses in Figure 8) covered a wider, less-centered range in step length and velocity than the distribution of Free Walk 2 (circles in Figure 8). Moreover, certain seismograph step length values in Free Walk 1 were either over- or underestimated compared with the pressure-sensitive walkway. The other three ambulation experiments (i.e., Free Walk 2, Swift Walk, and Normal Walk) exhibited matching distributions.

The outcomes of the paired two-sample t-tests revealed no statistically significant difference between the gait parameters measured with the pressure-sensitive walkway and those measured with seismographs (Table 1).

**Table 1**

| Gait Parameter | Walkway | | Seismograph | | t(200) | p | d |
|---|---|---|---|---|---|---|---|
| | M | SD | M | SD | | | |
| Step Time (s) | 0.604 | 0.092 | 0.603 | 0.092 | -1.583 | 0.115 | 0.008 |
| Cycle Time (s) | 1.206 | 0.186 | 1.205 | 0.185 | -0.556 | 0.579 | 0.003 |
| Ambulation Time (s) | 4.206 | 0.737 | 4.199 | 0.737 | -1.528 | 0.128 | 0.011 |
| Cadence (min⁻¹) | 101.23 0 | 13.780 | 101.378 | 13.620 | 0.893 | 0.373 | 0.011 |
| Velocity (cm/s) | 103.03 5 | 16.603 | 102.987 | 16.529 | -0.168 | 0.867 | 0.003 |
| Step Length (cm) | 60.997 | 3.918 | 60.737 | 4.200 | -1.436 | 0.153 | 0.064 |

Furthermore, for all evaluated gait parameters, similar mean, and standard deviation values were found with small associated effect sizes (d < 0.2). Likewise, upon categorizing the measured individuals based on age (e.g., those above 40 years and those 40 years or younger), there was no significant difference in the gait analysis results obtained from both the pressure-sensitive walkway and the seismographs. Comparable outcomes were also found when different age groups were formed. A significant, strong positive correlation with small standard errors for all analyzed gait parameters was found between the measurements obtained with seismographs and those obtained with the pressure-sensitive walkway (Table 2).

**Table 2**

| Gait Parameter | *r*(200) | *SE* | 95% CI | | *p* |
|---|---|---|---|---|---|
| | | | LL | UL | |
| Step Time (s) | 0.996 | 0.006 | 0.996 | 0.998 | < 0.001 |
| Cycle Time (s) | 0.996 | 0.005 | 0.996 | 0.997 | < 0.001 |
| Ambulation Time (s) | 0.995 | 0.007 | 0.994 | 0.996 | < 0.001 |
| Cadence (min⁻¹) | 0.985 | 0.012 | 0.981 | 0.989 | < 0.001 |
| Velocity (cm/s) | 0.971 | 0.017 | 0.962 | 0.978 | < 0.001 |
| Step Length (cm) | 0.804 | 0.039 | 0.749 | 0.848 | < 0.001 |

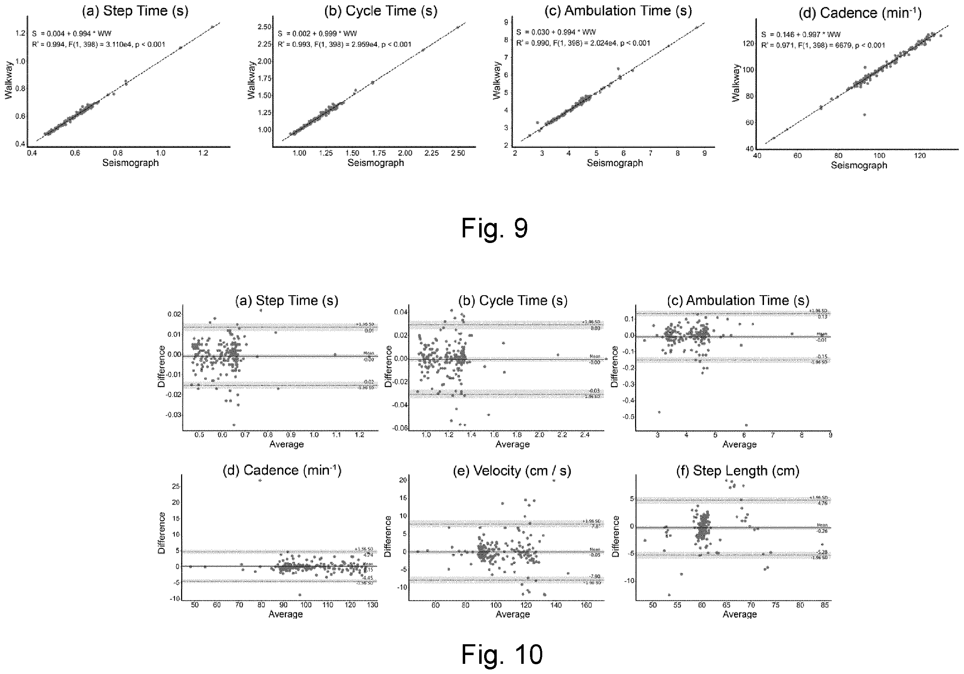

The Pearson correlation coefficients were above 0.970, with the exception of step length, which exhibited a Pearson correlation coefficient of 0.804. Furthermore, the analysis revealed that for all assessed gait parameters, with the exception of step length, both the lower and upper bounds of the 95% confidence intervals were high (LL > 0.950 and UL > 0.975). Conversely, the confidence interval for step length ranged from 0.749 to 0.848, indicating a lower degree of correlation relative to the other parameters. The F-statistics showed significant linear relationships (p < 0.001) with slopes close to one between gait parameters measured using the seismographs and the pressure-sensitive walkway (Figure 9). In this context, high coefficients of determination (i.e., R2 > 0.9) were found for the analyzed gait parameters except for step length, which exhibited a moderate coefficient of determination (R2 = 0.626) (Figure 7). With respect to the step length, it is important to emphasize that the off-the-shelf system used in the study was operating at a sampling rate of 100 Hz. As a consequence, the localization method based on the triangulation of the determined step event was less accurate with respect to this parameter.

The Bland-Altman plots revealed an agreement between gait parameters measured by the pressure-sensitive walkway and the seismographs (Figure 10). The analysis identified negligible biases, indicating an absence of proportional bias and a near-zero constant bias. Furthermore, the limits of agreement, which encompass 95% of the discrepancies between the two sets of measurements, demonstrated acceptable values with only a few outliers. Additionally, the variation in differences across various means was uniformly low and consistent.

A total of 50 TUG tests were recorded. The TUG test timings for the camera system ranged between 5.338 s and 10.811 s, and those for the seismographs ranged between 5.110 s and 10.670 s. There was no significant difference in measured TUG times between the camera system (M = 8.303s, SD = 1.250s) and the seismographs (M = 8.301s, SD = 1.273s), t(50) = -0.038, p = 0.970. A significant linear relationship was also observed between timings measured with seismographs and the camera system (F(1, 98) = 1.045, p < 0.001), with a high coefficient of determination (R2 = 0.970) predictor. Finally, a strong positive correlation between the average camera system and seismograph timings was found (r(50) = 0.985, p < 0.001).

The present inventive method represents a novel method for deriving clinically relevant gait parameters from seismographic measurements. In line with the first hypothesis, the study provided evidence showing that the mean values of matching gait parameters derived from seismographic and pressure-sensitive walkway measurements exhibited no significant differences. Furthermore, the findings substantiated the second hypothesis, revealing that the TUG mobility test timings determined using the seismographs did not significantly deviate from those obtained using the camera system.

The results of the present study confirmed the effectiveness of integrating mathematical morphologies with CTF for isolating seismic events of interest (i.e., footsteps) while mitigating low-frequency noise in vibration signals. Compared to bandpass filtering, the CTF method exhibits greater resilience against the frequency mixing issue, where seismic signals and noise overlap in frequency bands. Additionally, CTF demonstrated great potential in diminishing low-frequency noise compared to both white and black top-hat transformations.

Although damping slightly weakened the strength of measured seismic signals, the effectiveness of the present step extraction method remained unaffected. This robustness can be attributed to a combination of strategic approaches within the present methodology. Firstly, the identification of footstep events was based exclusively on the timestamp of peaks rather than their amplitude. Secondly, peaks produced by applying the attenuation stage exhibited distinct characteristics compared to non-peaks. Lastly, aggregation strategies were implemented, including the maximum combination of seismic signals measured by the three seismographs, to enhance any diminished amplitudes, effectively counteracting potential negative effects on signal quality. The findings of the present study demonstrated that even with the use of just one seismograph, the algorithm was capable of successfully identifying nearly 99% of all performed steps.

The comparison between gait parameters - including step time, cycle time, ambulation time, cadence, speed, and step length-measured with seismographs and the pressure-sensitive walkway showed a linear relationship with strong positive correlations. Additionally, the distributions of velocity and step length obtained through the seismograph closely mirrored those obtained through the pressure-sensitive walkway. Given that such distributions have been previously identified as indicators of health changes in older adults, the present results imply that seismographs can effectively function as a reliable digital tool for monitoring these health variations.

Gait parameters that were computed based on the temporal detection of two consecutive steps showed a high agreement between the two systems, and thus, systematic errors in step detection did not have an impact on them and were consistent across consecutive steps.

The statistical analysis conducted between the seismographs and the camera system showed a strong positive correlation and a high accuracy in assessing the timings of the TUG test. These findings indicate that the seismograph-based method can help to automate the evaluation of timed mobility tests, ultimately leading to a more objective medical assessment. Notably, an accurate assessment of these timing results was achieved without intrusive installation or calibration of the sensing devices. Considering the cost-effectiveness of seismographs, the developed method is not only promising as an ambient technology but also as a reliable solution for ongoing home monitoring of older adults.

It should be pointed out that the foregoing has outlined pertinent non-limiting embodiments. It will be clear to those skilled in the art that modifications to the disclosed non-limiting embodiments can be effected without departing from the spirit and scope thereof. As such, the described non-limiting embodiments ought to be considered merely illustrative of some of the more prominent features and applications. Other beneficial results can be realized by applying the non-limiting embodiments in a different manner or modifying them in ways known to those familiar with the art.

## Claims

1. A method (100) for measuring at least one spatial, temporal or spatiotemporal gait parameter, comprising:
a. Acquiring seismic signals by at least three time-synchronized seismographs positioned to span the floor of a measurement zone during walking of at least one walker on the floor of the measurement zone (101);
b. Receiving and processing the seismic signals by an analysis device to obtain a combined signal representing an aggregate signal of the seismographs (102);
c. Identifying and extracting footsteps events from the seismic combined signal, wherein each footstep event corresponds to the contact between a walker's foot and the floor in the measurement zone (103);
d. Calculating at least one gait parameter from the combined signal and the extracted footstep events (104).

2. The method according to claim 1, wherein the at least one measured gait parameter is selected from: step count, step time, stride time, cycle time, stance time, swing time, ambulation time, walking time, cadence, velocity, speed, footedness step length, stride length, walking distance, gait symmetry, and walking direction.

3. The method according to claim 1 or 2, further comprising temporally aligning the signals from the seismographs for generating the combined signal.

4. The method according to any one of the preceding claims, further comprising localization of footstep events by comparing signal arrival times at the seismographs.

5. The method according to claim 4, wherein localization of footstep event is determined by using an estimation of the wave propagation velocity in the measurement zone, preferably wherein the estimation of the wave propagation velocity in the measurement zone is based on a calibration step.

6. The method according to any one of the preceding claims, further comprising applying morphological filtering to the combined signal for noise reduction.

7. The method according to any one of the preceding claims, further comprising determining an average walking direction between footstep events by calculating a walking direction vector based on location and time differences of the footstep events.

8. The method according to claim 8, wherein footedness is determined by comparing footstep event locations with the average walking direction.

9. The method according to any of the preceding claims, further comprising calculating for each footstep event a time difference between the contact of the heel of the walker and the floor and the contact between the toe of the walker and the floor.

10. The method according to any one the preceding claims, further comprising extracting a signature signal for each walker identifying the walker and storing signature signals in a database.

11. The method according to claim 10, wherein at least two walkers are present in the measurement zone, and the combined signal is decomposed into person-specific signals using signature signals to measure walker-specific gait parameters.

12. The method according to any one of the preceding claims, further comprising calculating walking energy for each footstep event by combining the signals of the corresponding footstep event acquired by the at least three seismographs.

13. A system for measuring at least one spatial, temporal or spatiotemporal gait parameter (200), comprising:
• At least three seismographs (201) positioned to span the floor of a measurement zone (202) and configured to acquire seismic signals during walking of at least one walker in the measurement zone;
• An analysis device (203) coupled to receive the seismic signals from the seismographs and configured to:
• Process the received seismic signals to obtain a combined signal representing the aggregate signal of the seismographs (201); and
• Identify and extract footsteps events from the combined signal, wherein each footstep event corresponds to the contact between a walker's foot and the floor in the measurement zone (202);
• A processor (204) configured to calculate at least one, spatial, temporal or spatiotemporal gait parameter from the combined signal and the extracted footstep events; and
• A memory device (205) coupled to store the calculated gait parameters.

14. The system according to claim 14, wherein the analysis device is further configured to apply morphological filtering to the combined signal for noise reduction.

15. The system according to any of claims 14 or 15, wherein the processing unit is further configured to determine an average walking direction between footstep events and calculate footedness based on location differences.
